# EUROPEAN PATENT SPECIFICATION

(11) **EP 3 458 060 B1**
(45) Date of publication and mention of the grant of the patent: **26.06.2024**
(21) Application number: 17800027.9
(22) Date of filing: 16.05.2017
(51) Int. Cl.: A61K 31/475, A61K 31/4985, A61P 15/00, A61K 31/505, A61K 31/495, A61K 31/343, A61K 9/20, A61K 45/06, A61K 9/00, A61K 31/18, A61P 15/02, A61P 15/10, A61P 15/12

(54) **COMBINATION THERAPY FOR MALE SEXUAL DYSFUNCTION**
KOMBINATIONSTHERAPIE BEI MÄNNLICHER SEXUELLER DYSFUNKTION
POLYTHÉRAPIE POUR UN DYSFONCTIONNEMENT SEXUEL CHEZ L'HOMME

(30) Priority: 16.05.2016 US 201662336797 P
(43) Date of publication of application: 27.03.2019
(73) Proprietor: Foster, Howell, Little Rock, Arkansas 72210 (US)
(72) Inventor: Foster, Howell, Little Rock, Arkansas 72210 (US)
(74) Representative: J A Kemp LLP
(86) International application number: PCT/US2017/032942
(87) International publication number: WO 2017/201071

(56) References cited:
- EP-A1- 2 316 435
- US-A1- 2006 094 704
- MARRISSA MARTYN-ST JAMES ET AL: "Phosphodiesterase Type 5 Inhibitors for Premature Ejaculation: A Systematic Review and Meta-analysis", EUROPEAN UROLOGY FOCUS, vol. 3, no. 1, 1 February 2016 (2016-02-01), pages 119-129, XP055645065, NL ISSN: 2405-4569, DOI: 10.1016/j.euf.2016.02.001
- HAM WON SIK ET AL: "Recent Concepts of Premature Ejaculation", KOREAN JOURNAL OF UROLOGY, vol. 49, no. 9, 1 January 2008 (2008-01-01), pages 765-774, XP055236793, DOI: 10.4111/kju.2008.49.9.765
- GUR SERAP ET AL: "Current therapies for premature ejaculation", DRUG DISCOVERY TODAY, ELSEVIER, AMSTERDAM, NL, vol. 21, no. 7, 11 May 2016 (2016-05-11), pages 1147-1154, XP029598211, ISSN: 1359-6446, DOI: 10.1016/J.DRUDIS.2016.05.004
- E. C. POLAT ET AL: "Combination therapy with selective serotonin reuptake inhibitors and phosphodiesterase-5 inhibitors in the treatment of premature ejaculation", ANDROLOGIA., vol. 47, no. 5, 9 May 2014 (2014-05-09), pages 487-492, XP055442675, DE ISSN: 0303-4569, DOI: 10.1111/and.12289
- POLAT EC et al.: "Combination therapy with selective serotonin reuptake inhibitors and phosphodiesterase-5 inhibitors in the treatment of premature ejaculation", Andrologia, vol. 47, no. 5, June 2015 (2015-06), pages 487-492, XP055442675,
- AKIN Y et al.: "Comparison of alpha blockers in treatment of premature ejaculation: a pilot clinical trial", Iran Red Crescent Med J., vol. 15, no. 10, 2013, pages 1-5, XP055442679,
- SAFARINEJAD MR: "Safety and efficacy of escitalopram in the treatment of premature ejaculation: a double-blind, placebo-controlled, fixed-dose, randomized study", J Clin Psychopharmacol., vol. 27, no. 5, October 2007 (2007-10), pages 444-50, XP009514577, DOI: 10.1097/jcp.0b013e31814b98d4
- HATZIMOURATIDIS K.(CHAIR et al.: "Guidelines on Male Sexual Dysfunction: Erectile dysfunction and premature ejaculation", European Association of Urology, March 2015 (2015-03), pages 1-28, XP055442683,

## Description

### Field

The invention relates to compositions useful in the treatment of premature ejaculation. The invention relates to co-formulations containing a serotonin reuptake inhibitor and a phosphodiesterase 5 inhibitor as on-demand formulations to increase ejaculatory latency time. In particular, the present invention provides a pharmaceutical composition for use in delaying onset of ejaculation by a patient in need thereof.

### Related Art

Premature ejaculation (PE) is one of the most common forms of male sexual dysfunction. By some estimates, as many as one in three males have complained of PE at some point in their lives. PE is commonly defined as ejaculation before, shortly after, or upon penetration, and before the man wishes. The most common clinical definition of PE is ejaculation within one minute of penetration. PE may be lifelong, starting with first sexual experience (primary) or acquired later (secondary). According to the International Society for Sexual Medicine (ISSM), ejaculation which always or nearly always occurs prior to or within about one minute of vaginal penetration from the first sexual experience (lifelong premature ejaculation) is primary PE and is considered to be genetic. A clinically significant reduction in latency time, often to about 3 minutes or less after penetration is labeled by the ISSM as acquired premature ejaculation (a.k.a. secondary or variable PE).

Various regimens have been proposed and used for the treatment of PE. Those treatments include behavioral, topical, and systemic medicaments. The effectiveness of PE treatment is generally assessed by measuring positive changes in intravaginal ejaculatory latency time (IELT). Other measures of effectiveness include improved sexual satisfaction, control over ejaculation, relationship satisfaction, self-esteem, and quality of life. As with any medical intervention, the positive benefits are balanced against side effects and safety concerns.

Those medical interventions that demonstrate some improvement in IELT include topical anesthetics, selective serotonin reuptake inhibitors (SSRIs), serotonin-noradrenaline reuptake inhibitors (SNRIs), tricyclic antidepressants, phosphodiesterase-5 inhibitors (PDE5i), opioid- based analgesics *(e.g.,* tramadol), α-blockers *(e.g.,* selective α1-adrenoceptor antagonists), acupuncture, devices, and behavioral therapies - including stop-start, the squeeze technique and yoga.

Tricyclic antidepressants were assessed for their ability to treat PE as early as 1973 (see H. Eaton, "Clomipramine (Anafranil) in the treatment of premature ejaculation," J. Int. Med. Res. 1973; 1:432-4). Clomipramine orally administered daily at 10 to 50 mg per day has been shown to effectively increase IELT (Rowland et al., "Effective daily treatment with clomipramine in men with premature ejaculation when 25 mg (as required) is ineffective," BJU Int. 2001 Mar; 87(4):357- 6; R. Goodman, "An assessment of clomipramine (Anafranil) in the treatment of premature ejaculation," J Int Med Res. 1980; Segraves et al., "Clomipramine versus placebo in the treatment of premature ejaculation: a pilot study," J. Sex. Marital. Ther. 8 Suppl 3:53-9; 1993 Fall). Inhaled (nasal) clomipramine (4 mg or 5 mg) administered on-demand *(e.g.,* one hour pre-coitus) has also been shown to be effective in treating PE, but with the unwanted effect of nasal irritation.

As early as 1994, SSRIs have been investigated for the treatment of PE (see P. Kaplan, "The use of serotonergic uptake inhibitors in the treatment of premature ejaculation," J. Sex. Marital. Ther. 1994 Winter; 20(4):321-4). SSRIs are generally administered daily at dosages of 10 mg to 60 mg per day and in some cases may take several weeks to effectively relieve symptoms of depression. SSRIs include citalopram, escitalopram, fluoxetine, paroxetine, sertraline, and dapoxetine. The SNRI duloxetine has also been shown to have some positive effect on IELT.

PDE5 inhibitors were investigated for their effect on PE as early as 2001 (Abdel-Hamid et al., "Assessment of as needed use of pharmacotherapy and the pause-squeeze technique in premature ejaculation," Int. J, Impotence Research. (2001) 13, 41-45). PDE5 inhibitors include avanafil, lodenafil, mirodenafil, sildenafil, tadalafil, vardenafil, udenafil, zaprinast, icariin and synthetic derivatives thereof, and benzamidenafil. For example, sildenafil was administered at 50 mg pre-coitus and, while no better than placebo when administered alone, it was shown to increase IELT to about 3.63 minutes when combined with behavioral psychotherapy (Tang et al., "Clinical efficacy of Viagra with behavior therapy against premature ejaculation," Zhonghua Nan Ke Xue 2004;10:366-7).

Combinations of various therapies have been explored for the treatment of PE. In 2002, Salonia *et al.* conducted a prospective study comparing the effect of a PDE5i alone and a PDE5i combined with an SSRI on IELT (Salonia et al., "A prospective study comparing paroxetine alone versus paroxetine plus sildenafil in patients with premature ejaculation," 2002, Journal Urology, 168, 2486-2489). In one study, dapoxetine (30 mg) plus microdenafil (50 mg) were administeredon demand *(i.e.,* 1-3 hours pre-coitus) (Lee et al., "Comparison between on-demand dosing of dapoxetine alone and dapoxetine plus mirodenafil in patients with premature ejaculation; prospective, randomized, double-blind, and placebo-controlled," J Sex Med 2012;9:203-4). In another study, fluoxetine (90 mg) was administered once per week, and tadalafil (20 mg) was administered on demand (Mattos et al., "Tadalafil and fluoxetine in premature ejaculation: prospective, randomized, double-blind, placebo-controlled study," Urol Int 2008;80:162-5). In another study, fluoxetine (20 mg) was administered on a daily basis, and fluoxetine (20 mg) two to three hours pre-coitus plus sildenafil (50 mg) one hour pre-coitus (Hosseini and Yarmohammadi, "Effect of fluoxetine alone and in combination with sildenafil in patients with premature ejaculation," Urol Int. 2007;79(1):28-32H). In yet another study, sertraline (50 mg) was administered on a daily basis, and sildenafil (50 mg) was administered one hour pre-coitus (Zhang et. Al., "Comparison between sildenafil plus sertraline and sertraline alone in the treatment of premature ejaculation," Zhonghua Nan Ke Xue 2005;11:520-5).

While some progress has been made in the development of safe and effective treatments for PE, there still remains a significant unmet medical need for a reliable, effective, and convenient on demand treatment for PE. The inventor has made the surprising discovery that a pharmaceutical formulation containing escitalopram and tadalafil, as an on-demand form, effectively increases IELT and improves overall sexual well-being of the subject.
The following documents are mentioned:
- EP2316435 which refers to pharmaceutical compositions of PDE-5 inhibitors and dapoxetine;
- Martyn-St James et al (2017) European Urology Focus, 3(1): 119-129 which refers to phosphodiesterase type-5 inhibitors for premature ejaculation: a systematic review and meta-analysis;
- Ham et al (2008) Korean Journal of Urology, 49(9): 765-774 which refers to recent concepts of premature ejaculation;
- Gur et al (2016) Drug Discovery Today, 21(7): 1147-1154 which refers to current therapies for premature ejaculation.
- Polat et al (2014) Andrologia, 47(5): 487-492 which refers to combination therapy with selective serotonin reuptake inhibitors and phosphodiesterase-5 inhibitors in the treatment of premature ejaculation.

### SUMMARY

The invention provides pharmaceutical formulation for use within 36 hours before coitus, 2 hours to about 24 hours before coitus, or 30 minutes to about 5 hours before coitus to delay onset of ejaculation by a patient in need thereof comprising tadalafil, escitalopram, and one of more pharmaceutically acceptable excipients.. The drug agents include the non-salt form of the drug, enantiomers of the drug, and salts of the drug. The references to methods of treatment in the subsequent paragraphs of this description are to be interpreted as references to the compounds, pharmaceutical compositions and medicaments of the present invention for use in a method for treatment by therapy.

The formulations of the invention comprise tadalafil as a PDE5i. In some embodiments, the PDE5i is tadalafil and any one or more of avanafil, lodenafil, mirodenafil, sildenafil, vardenafil, udenafil, zaprinast, icariin and synthetic derivatives thereof, and benzamidenafil. In other embodiments, the formulation is one excluding mirodenafil and sildenafil as a PDE5i.

In the invention, the SRI, in particular SSRI or SNRI, is escitalopram. In some embodiments, the SSRI or SNRI is escitalopram and any one or more of citalopram, fluoxetine, paroxetine, sertraline, dapoxetine, seproxetine, zimelidine, mesembrine, vilazodone, venlafaxine, duloxetine, desvenlafaxine, and milnacipran. In other embodiments, the formulation is one excluding dapoxitine, fluoxetine, and sertraline as a SSRI or SNRI.

In some embodiments, about one to about 30 milligrams (mg) of each drug agent is present in a combined dosage form of the pharmaceutical formulation. In one embodiment, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, about 15 mg, about 15.5 mg, about 16 mg, about 16.5 mg, about 17 mg, about 17.5 mg, about 18 mg, about 18.5 mg, about 19 mg, about 19.5, or about 20 mg of PDE5i is present in a combined dosage form. In a specific embodiment, the PDE5i is tadalafil, which is present in a combined dosage form at about 4 mg, about 5 mg, about 6.9 mg, about 9 mg, or about 13.8 mg.

In one embodiment, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, about 15 mg, about 15.5 mg, about 16 mg, about 16.5 mg, about17 mg, about 17.5 mg, about 18 mg, about 18.5 mg, about 19 mg, about 19.5 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, or about 40 mg of SRI is present in a single dosage form. In a specific embodiment, the SRI is escitalopram, which is present in a single dosage form at about 4 mg, about 5 mg, about 9.6 mg, about 18 mg, or about 19.2 mg.

Also disclosed is a combined dosage form is formulated as a once daily form. Here, the SRI, namely escitalopram, and the PDE5i, namely tadalfil, are each present in the single dosage form at about 4 mg or at about 5 mg. In a preferred instance of the claimed invention, the SRI namely escitalopram, and the PDE5i namely tadalafil, are each present in the single dosage form at about 18 mg and 9 mg, respectively.

The pharmaceutical formulation of the present invention is one for use within 36 hours before coitus, 2 hours to about 24 hours before coitus, or 30 minutes to about 5 hours before coitus to delay onset of ejaculation by a patient in need thereof. The combined dosage form is formulated as an on-demand form. In some embodiments, the on-demand dosage form may be taken or administered about 2 hours to about 24 hours before coitus. In some embodiments, the on-demand dosage form may be taken or administered about 30 minutes to about 5 hours before coitus. In some embodiments, the on- demand dosage form may be taken or administered about 30 minutes, about 40 minutes, about 50 minutes, about 60 minutes, about 70 minutes, about 80 minutes, about 90 minutes, about 100 minutes, about 110 minutes, about 120 minutes, about 2.5 hours, about 3 hours, about 3.5 hours, about 4 hours, about 4.5 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, or about 36 hours prior to coitus.

In a specific on-demand single dose formulation, the PDE5i, namely tadalafil, is present at about 6.9 mg and the SRI, namely escitalopram, is present at about 9.6 mg. In another specific on-demand single dose formulation, said PDE5i is present at about 6.9 mg and the SRI, namely escitalopram, is present at about 19.2 mg. In yet another specific on-demand single dose formulation, said PDE5i is present at about 13.8 mg and the SRI, namely escitalopram, is present at about 19.2 mg. In another specific on-demand single dose formulation, said PDE5i is present at about 9 mg and the SRI , namely escitalopram, is present at about 18 mg. In yet another specific on-demand single dose formulation, the PDE5i is present at about 5 mg and the SRI (escitalopram) is present at about 5 mg.

In one embodiment, the combined dose formulation is in the form of a tablet, a capsule, a liquid, a powder, a lyophilized cake, an oral film, troche, or an intranasal preparation. In one embodiment, the formulation is an oral disintegrating tablet.

Also disclosed but not part of the claimed subject-matter is a pharmaceutical formulation comprising at least two drug agents, (i) an alpha-blocker (i.e., a neutral antagonist or inverse agonist of alpha- adrenergic receptors) and (ii) a serotonin reuptake inhibitor (SRI), combined together in a single dosage form. SRI includes selective serotonin reuptake inhibitors (SSRIs) and serotonin- norepinephrine reuptake inhibitors ( SNRIs). The drug agents include the non-salt form of the drug, enantiomers of the drug, and salts of the drug.

In some instances, not part of the claimed subject-matter the alpha-blocker is any one or more of tamsulosin, doxazosin, prazosin, terazosin, alfuzosin, phentolamine, carvedilol, silodosin, phenoxybenzamine, dutasteride/tamsulosin, tamsulosin hydrochloride, terazosin hydrochloride, alfuzosin hydrochloride, prazosm hydrochloride, phentolamine mesylate, phenoxybenzamine hydrochloride, parvedilol phosphate, perazosin hydrochloride anhydrous, and poxazosin mesylate.

In some instances, not part of the claimed subject-matter the SSRI or SNRI is any one or more of citalopram, escitalopram, fluoxetine, paroxetine, sertraline, dapoxetine, seproxetine, zimelidine, mesembrine, vilazodone, venlafaxine, duloxetine, desvenlafaxine, and milnacipran,. In other instances, the SSRI or SNRI is any SSRI or SNRI, excluding dapoxitine, fluoxetine, and sertraline. In the invention the SSRI or SNRI is escitalopram.

In one instance, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, about 15 mg, about 15.5 mg, about 16 mg, about 16.5 mg, about 17 mg, about 17.5 mg, about 18 mg, about 18.5 mg, about 19 mg, about 19.5 mg, or about 20 mg of SRI is present in a single dosage form. In a specific embodiment of the invention, the SRI is escitalopram, which is present in a single dosage form at about 4 mg, about 5 mg, about 9.6 mg, about 18 mg, or about 19.2 mg.

In one instance, not part of the claimed subject-matter, about 0.05mg, about 0.1 mg, about 0.15 mg, about 0.2 mg, about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, about 1.0 mg, about 1.5 mg, about 1.6 mg, about 1.7 mg, about 1.8 mg, about 1.9 mg, or about 2 mg of alpha-blocker is present in a combined dosage form. In a specific instance, the alpha-blocker is tamsulosin, which is present in a combined dosage form at about 0.2 mg, about 0.4 mg, about 0.6 mg, or about 0.8 mg.

Also disclosed, but not part of the claimed subject-matter, is a specific on-demand single dose formulation, where the alpha-blocker is present at about 0.2mg and the SRI (e.g., escitalopram) is present at about 9.6 mg. In another specific on-demand single dose formulation, the alpha-blocker is present at about 0.4 mg and the SRI (e.g., escitalopram) is present at about 19.2 mg. In yet another specific on-demand single dose formulation, the alpha-blocker is present at about 0.8 mg and the SRI (e.g., escitalopram) is presentat about 19.2 mg. In yet another specific on-demand single dose formulation, the alpha-blocker ispresent at about 0.8 mg and the SRI (e.g., escitalopram) is present at about 18 mg.

In some instances, the combined dosage form is formulated as an on-demand form. In some instances, the on-demand dosage form may be taken or administered about 2 hours to about 24 hours before coitus. In some instances, the on-demand dosage form may be taken or administered about 30 minutes to about 5 hours before coitus. In some instances, the on-demand dosage form may be taken or administered about 30 minutes, about 40 minutes, about 50 minutes, about 60 minutes, about 70 minutes, about 80 minutes, about 90 minutes, about 100 minutes, about 110 minutes, about 120 minutes, about 2.5 hours, about 3 hours, about 3.5 hours, about 4 hours, about 4.5 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, or about 36 hours prior to coitus.

Also disclosed, but not part of the claimed subject-matter, is a specific on-demand single dose formulation, the alpha-blocker is present at about 0.2mg and the SRI (e.g., escitalopram) is present at about 9.6 mg. In another specific on-demand single dose formulation, the alpha-blocker is present at about 0.4 mg and the SRI (e.g., escitalopram) is present at about 19.2 mg. In yet another specific on-demand single dose formulation, the alpha-blocker is present at about 0.8 mg and the SRI (e.g., escitalopram) is present at about 19.2 mg. In yet another specific on-demand single dose formulation, the alpha blocker ispresent at about 0.8 mg and the SRI (e.g., escitalopram) is present at about 18 mg.

In one instance, the combined dose formulation, whether the on-demand formulation or the daily dose formulation, is in the form of a tablet, a capsule, a liquid, a powder, a lyophilized cake, an oral film, troche, or an intranasal preparation. In one instance, the formulation is an oral disintegrating tablet.

**[Deleted]**

In one embodiment the pharmaceutical formulation of the present invention is for use in a method of treating premature ejaculation in a subject in need thereof, by co-administering to the subject the tadalafil, and escitalopram. In some embodiments, the tadalafil, and escitalopram are combined in a single dosage form. In other embodiments, the tadalafil, and escitalopram are administered approximately contemporaneously to the subject in separate formulations..

On-demand means that the drug agents are taken or administered within 36 hours, within about 35 hours, within about 34 hours, within about 33 hours, within about 32 hours, within about 31 hours, within about 30 hours, within about 29 hours, within about 28 hours, within about 27 hours, within about 26 hours, within about 25 hours, within about 24 hours, within about 23 hours, within about 22 hours, within about 21 hours, within about 20 hours, within about 19 hours, within about 18 hours, within about 17 hours, within about 16 hours, within about 15 hours, within about 14 hours, within about 13 hours, within about 12 hours, within about 11 hours, within about 10 hours, within about 9 hours, within about 8 hours, within about 7 hours, within about 6 hours, within about 5 hours, within about 4 hours, within about 3 hours, within about 2 hours, within about 90 minutes, within about 1 hour, or within about 30 minutes of coitus.

In some instances, the PDE5i is a drug agent that selectively inhibits phosphodiesterase 5 at a lower IC50 than its IC50 for other phosphodiesterases. In one embodiment, the PDE5i has a PDE5 IC50 that is at least about 100-fold lower, at least about 200-fold lower, at least about 300- fold lower, at least about 400-fold lower, at least about 500-fold lower, at least about 600-fold lower, at least about 700-fold lower, at least about 800-fold lower, at least about 900-fold lower, or at least about 1000-fold lower than its IC50 for another phosphodiesterase.

In some instances, the PDE5i has a half-life of between about 4 hours and 24 hours or between 15 hours and 24 hours. In one embodiment, the PDE5 l has a half-life of ≥ 4 hours, ≥ 5 hours, ≥ 6 hours, ≥ 7 hours, ≥ 8 hours, ≥ 9 hours, ≥ 10 hours, ≥ 11 hours, ≥ 12 hours, about 13 hours, about 14 hours, about 15 hours, about 16 hours, about 17 hours, about 18 hours, about 19 hours, about 20 hours, about 21 hours, about 22 hours, about 23 hours, or about 24 hours.

The pharmaceutical formulation of the present invention comprises tadalafil as a PDE5i. In some embodiments, the PDE5i comprises tadalafil and any one or more of avanafil, lodenafil, mirodenafil, sildenafil, vardenafil, udenafil, zaprinast, icariin and synthetic derivatives thereof, and benzamidenafil. In other embodiments, the PDE5i excludes mirodenafil and sildanafil. In a specific embodiment, the PDE5i is tadalafil, a therapeutically effective enantiomer of tadalafil, or a salt of tadalafil.

The pharmaceutical formulation of the present invention comprises escitalopram as a SRI. In some embodiments, the SRI is a drug agent that selectively inhibits serotonin reuptake at the synapse *(i.e.,* an SSRI). In some embodiments, the SRI has a half-life of between about 15 hours and 4 days. In some embodiments, the half-life of the SRI is less than about 40 hours, less than about 35 hours, less than about 30 hours, less than about 25 hours, less than about 20 hours, or less than about 15 hours.

In some embodiments, the SRI comprises escitalopram and any one or more of citalopram, escitalopram, fluoxetine, paroxetine, sertraline, dapoxetine, seproxetine, zimelidine, mesembrine, vilazodone, venlafaxine, duloxetine, desvenlafaxine, and milnacipran. In other embodiments, the SRI excludes dapoxitine, fluoxetine, and sertraline. In a specific embodiment, the SRI is escitalopram, a therapeutically effective enantiomer of escitalopram *(e.g.*, citalopram), or a salt of escitalopram.

In one embodiment, premature ejaculation encompasses ejaculation that occurs before penetration, upon initial penetration, or shortly after penetration. In another embodiment, premature ejaculation encompasses ejaculation that occurs with minimal stimulation. In another embodiment, premature ejaculation encompasses ejaculation that occurs before the subject or before the subject's partner wishes. In yet another embodiment, premature ejaculation encompasses ejaculation that occurs within about 60 seconds, within about 90 seconds, within about 2 minutes, within about 2.5 minutes, or within about 3 minutes of initial penetration.

In one embodiment, premature ejaculation comprises a lifelong condition, *i.e.*, primary *ejaculatio praecox*, or a condition acquired later in life, i.e., secondary *ejaculatio praecox.*

In some embodiments, the pharmaceutical formulation is one for use where about one to about 30 milligrams (mg) of each drug agent is administered to the subject per dose. In one embodiment, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, about 15 mg, about 15.5 mg, about 16mg, about 16.5 mg, about 17 mg, about 17.5 mg, about 18 mg, about 18.5 mg, about 19 mg, about 19.5, or about 20 mg of PDE5i is administered in combination with or contemporaneously with the SRI. In the invention the PDE5i is tadalafil (which is administered in combination with or contemporaneously with the SRI) at about 4 mg, about 6.9 mg, about 9 mg, or about 13.8 mg per dose.

In one embodiment, the pharmaceutical formulation is one for use where about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, about 15 mg, about 15.5 mg, about 16 mg, about 16.5 mg, about 17 mg, about 17.5 mg, about 18 mg, about 18.5 mg, about 19 mg, about 19.5 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, or about 40 mg of SRI is administered in combination with or contemporaneously with the PDE5i. In the invention the SRI is escitalopram (which is administered in combination with or contemporaneously with the PDE5i) at about 4 mg, about 9.6 mg, about 18 mg, or about 19.2 mg per dose.

**[Deleted]**

**[Deleted]**

**[Deleted]**

The pharmaceutical formulation of the present invention is one for use within 36 hours before coitus, 2 hours to about 24 hours before coitus, or 30 minutes to about 5 hours before coitus to delay onset of ejaculation by a patient in need thereof comprising tadalafil, escitalopram, and one of more pharmaceutically acceptable excipients. The drug agents are administered to or taken by the subject on- demand. In some embodiments, on-demand means the drug agents are administered about 30 minutes to about 5 hours before coitus. In some embodiments, on-demand means the drug agents are administered about 30 minutes, about 40 minutes, about 50 minutes, about 60 minutes, about 70 minutes, about 80 minutes, about 90 minutes, about 100 minutes, about 110 minutes, about 120 minutes, about 2.5 hours, about 3 hours, about 3.5 hours, about 4 hours, about 4.5 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, or about 36 hours prior to coitus.

In specific on-demand administration regime, regimes according to the invention, about 6.9 mg PDE5i (tadalafil) is administered with about 9.6 mg SRI (escitalopram). In another specific on-demand administration regime, about 6.9 mg PDE5i (tadalafil) is administered with about 19.2 mg SRI *(e.g.*, escitalopram). In another specific on-demand administration regime, about 9 mg PDE5i (tadalafil) is administered with about 18 mg SRI (escitalopram). In yet another specific on-demand administration regime, about 13.8 mg PDE5i (tadalafil) is administered with about 19.2 mg SRI *(*escitalopram).

In one embodiment, the drug agents that are co-administered, contemporaneously administered, or administered in a single combined form, the drug(s) is /are formulated as a tablet, a capsule, a liquid, a powder, a lyophilized cake, an oral film, troche, or an intranasal preparation. In one embodiment, the drugs are formulated as an oral disintegrating tablet.

Also disclosed, but not part of the claimed invention, is a method of treating premature ejaculation in a subject in need thereof, by co-administering to the subject an alpha-blocker and a serotonin reuptake inhibitor (SRI). In some instances, the alpha-blocker and SRI are combined in a single dosage form. In other instances, the alpha-blocker and the SRI are administered approximately contemporaneously to the subject in separate formulations. In one instance, invention provides a method of treating premature ejaculation in a subject in need thereof, by co-administering to the subject an alpha-blocker, and a serotonin reuptake inhibitor (SRI). In some instances, the alpha-blocker and SRI are combined in a single dosage form. In other instances, the alpha-blocker and the SRI are administered approximately contemporaneously to the subject in separate formulations.

In some instances, not part of the claimed invention, the alpha-blocker and the SRI are administered daily, and in other embodiments, the alpha-blocker and SRI are administered on-demand. On-demand means that the drug agents are taken or administered within about 36 hours, within about 35 hours, within about 34 hours, within about 33 hours, within about 32 hours, within about 31 hours, within about 30 hours, within about 29 hours, within about 28 hours, within about 27 hours, within about 26 hours, within about 25 hours, within about 24 hours, within about 23 hours, within about 22 hours, within about 21 hours, within about 20 hours, within about 19 hours, within about 18 hours, within about 17 hours, within about 16 hours, within about 15 hours, within about 14 hours, within about 13 hours, within about 12 hours, within about 11 hours, within about 10 hours, within about 9 hours, within about 8 hours, within about 7 hours, within about 6 hours, within about 5 hours, within about 4 hours, within about 3 hours, within about 2 hours, within about 90 minutes, within about 1 hour, or within about 30 minutes of coitus.

In some instances, not part of the claimed invention, the alpha-blocker is a drug agent that antagonizes α-adrenoceptors and relaxes smooth muscle cells in the ureter, vas deferens, uterus, urethral sphincter, bronchioles, and blood vessels. In some instances, the alpha-blocker has a half-life of between about 8 hours and 1 day. In some instances, the alpha-blocker has a half-life of between about 9 and 13 hours. In some instances, the half-life of the alpha-blocker is less than about 24 hours, less than about 20 hours, less than about 18 hours, less than about 16 hours, less than about 15 hours, less than about 14 hours, less than about 13 hours, less than about 12 hours, less than about 11 hours, less than about 10 hours, less than about 9 hours, or less than about 8 hours. In one embodiment, the alpha-blocker is an alpha-2-blocker. In one instance, the alpha-blocker is an alpha-1-blocker. In one instance, the alpha-blocker is a selective α1A receptor antagonist.

In some instances, not part of the claimed invention, the alpha-blocker is any one or more of tamsulosin, doxazosin, prazosin, terazosin, alfuzosin, phentolamine, carvedilol, silodosin, phenoxybenzamine, dutasteride/tamsulosin, tamsulosin hydrochloride, terazosin hydrochloride, alfuzosin hydrochloride, prazosm hydrochloride, phentolamine mesylate, phenoxybenzamine hydrochloride, parvedilol phosphate, perazosin hydrochloride anhydrous, and poxazosin mesylate. In a preferred instance, the alpha-blocker is tamsulosin.

In some instances, the SRI is a drug agent that selectively inhibits serotonin reuptake at the synapse (i.e., an SSRI). In some instances, the SRI has a half-life of between about 15 hours and 4 days. In some instances, the half-life of the SRI is less than about 40 hours, less than about 35 hours, less than about 30 hours, less than about 25 hours, less than about 20 hours,or less than about 15 hours.

In some instances, the SRI comprises any one or more of citalopram, escitalopram, fluoxetine, paroxetine, sertraline, dapoxetine, seproxetine, zimelidine, mesembrine, vilazodone, venlafaxine, duloxetine, desvenlafaxine, and milnacipran. In other instances, the SRI is any SRI, excluding dapoxitine, fluoxetine, and sertraline. In the invention, the SRI is escitalopram, a therapeutically effective enantiomer of escitalopram (e.g., citalopram), or a salt of escitalopram.

In one embodiment, premature ejaculation encompasses ejaculation that occurs before penetration, upon initial penetration, or shortly after penetration. In another embodiment, premature ejaculation encompasses ejaculation that occurs with minimal stimulation. In another embodiment, premature ejaculation encompasses ejaculation that occurs before the subject or before the subject's partner wishes. In yet another embodiment, premature ejaculation encompasses ejaculation that occurs within about 60 seconds, within about 90 seconds, within about 2 minutes, within about 2.5 minutes, or within about 3 minutes of initial penetration.

In one embodiment, premature ejaculation comprises a lifelong condition, i.e., primary *ejaculatio praecox,* or a condition acquired later in life, i.e., secondary *ejaculatio praecox.*

In instance, not part of the claimed invention, about 0.05mg, about 0.1 mg, about 0.15 mg, about 0.2 mg, about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, about 1.0 mg, about 1.5 mg, about 1.6 mg, about 1.7 mg, about 1.8 mg, about 1.9 mg, or about 2 mg of alpha-blocker is present in a combined dosage form. In a specific instance, the alpha-blocker is tamsulosin, which is present in a combined dosage form at about 0.2 mg, about 0.4 mg, about 0.6 mg, or about 0.8 mg.

In one instance, not part of the claimed invention, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, about 15 mg, about 15.5 mg, about 16 mg, about 16.5 mg, about 18 mg, about 18.5 mg, about 19 mg, about 19.5 mg, about 20 mg, about 21 mg, about 22 mg, about 23 about mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, or about 40 mg of SRI is administered in combination with or contemporaneously with the alpha-blocker. In a specific instance, not part of the claimed invention, the SRI is escitalopram (which is administered in combination with or contemporaneously with the alpha- blocker) present at about 4 mg, about 9.6 mg, about 18 mg, or about 19.2 mg per dose.

In a specific instance, not part of the claimed invention, the drug agents are administered to or taken by the subject once daily. Here, about 18 mg of SRI (e.g., escitalopram) and about 0.4 mg of alpha-blocker (e.g., tamsulosin) are co-administered in a combined dosage form or administered contemporaneously as separate doses.

In some instances, the drug agents are administered to or taken by the subject on-demand. In some instances on-demand means the drug agents are administered about 30 minutes to about 5 hours before coitus. In some instances, on-demand means the drug agents are administered about 2 hours to about 24 hours before coitus. In some instances, on-demand means the drug agents are administered about 30 minutes, about 40 minutes, about 50 minutes, about 60 minutes, about 70 minutes, about 80 minutes, about 90 minutes, about 100 minutes, about 110 minutes, about 120 minutes, about 2.5 hours, about 3 hours, about 3.5 hours, about 4 hours, about 4.5 hours, about 5 hours, about 5.5 hours, about 6 hours, about 6.5 hours, about 7 hours, about 7.5 hours, about 8 hours, about 9 hours, about 10 hours, about 11 hours, about 12 hours, about 16 hours, about 20 hours, about 24 hours, about 28 hours, about 32 hours, or about 36 hours prior to coitus.

In a specific on-demand administration regime, not part of the claimed invention, about 0.4 mg alpha-blocker (e.g., tamsulosin) is administered with about 9.6 mg SRI (e.g., escitalopram). In another specific on- demand administration regime, about 0.4 mg alpha-blocker (e.g., tamsulosin) is administered with about 19.2 mg SRI (e.g., escitalopram). In yet another specific on-demand administration regime, about 0.4 mg or 0.8 mg alpha-blocker (e.g., tamsulosin) is administered with about 18 mg SRI (e.g., escitalopram).

In one instance, the drug agents that are co-administered, contemporaneously administered, or administered in a single combined form, the drug(s) is /are formulated as a tablet, a capsule, a liquid, a powder, a lyophilized cake, an oral film, troche, or an intranasal preparation.In one instance, the drugs are formulated as an oral disintegrating tablet.

In some embodiments, the intravaginal ejaculatory latency time (IELT) increases at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100% for the subject after co-administration of the drug agents or administration of the co-formulation, compared to the subject with no drug administered or only one drug *(i.e.,* either the PDE5i without the SRI, or the SRI without the PDE5i) administered. In some embodiments, the IELT increases at least about 15 seconds, at least about 30 seconds, at least about 45 seconds, at least about 60 seconds, at least about 75 seconds, at least about 90 seconds, at least about 105 seconds, at least about 2 minutes, or at least about 2½ minutes for the subject after co-administration of the drug agents or administration of the co-formulation, compared to the subject with no drug administered or only one drug administered.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about", when used in reference to a particular recited numerical value or range of values, means that the value may vary from the recited value by no more than 2%. For example, as used herein, the expression "about 100" includes 98 and 102 and all values in between (e.g., 98.00, 98.01, 98.02, 98.03, 98.04, ... , 101.96, 101.97, 101.98, 101.99, 102.00).

Although any methods and materials similar or equivalent to those described herein can be used in the practice or testing of the present invention, exemplar methods and materials are now described.

As used herein, the expression "pharmaceutical formulation" means a combination of at least one active ingredient (e.g., a small molecule, macromolecule, compound, etc. which is capable of exerting a biological effect in a human or non-human animal), and at least one inactive ingredient which, when combined with the active ingredient or one or more additional active or inactive ingredients, is suitable for therapeutic administration to a human or non-human animal. The term "formulation", as used herein, means "pharmaceutical formulation" unless specifically indicated otherwise. The present invention provides at least two pharmaceutical formulations containing one therapeutic small molecule that may be combined or co-administered to a subject. Also described are pharmaceutical formulations that contain at least two therapeutic small molecules. According to those embodiments of the present invention having at least two formulations, in one embodiment the therapeutic small molecule is a smooth muscle relaxant, which is a phosphodiesterase inhibitors comprising tadalafil, in one formulation, and a serotonin reuptake inhibitor in the other formulation which comprises escitalopram. According to those embodiments having a single formulation containing at least two therapeutic small molecules, the single formulation comprises tadalafil, escitalopram, and one of more pharmaceutically acceptable excipients.

Pharmaceutical compositions useful in the practice of the method of the invention include a therapeutically effective amount of an active agent, and a pharmaceutically acceptable carrier. The term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Suitable pharmaceutical excipients include starch, glucose, lactose, sucrose, other natural sweeteners, sugar alcohol, steviol glycosides, artificial sweeteners *(i.e.,* acesulfame potassium, aspartame, saccharin, and sucralose), natural and artificial flavorings, gelatin, malt, rice, flour, chalk, silica gel (micronized), sodium stearate, glycerol monostearate, talc, sodium chloride, dried skim milk, glycerol, propylene glycol, polyethylene glycol *(e.g.,* 3350), anticaking agents *(e.g.,* tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethylsiloxane), water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, rapidly dissolving oral (buccal) tablets and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin.

The active agents of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc. Pharmaceutically acceptable counter ions include *inter alia* hydrochloride, sodium, sulfate, acetate, phosphate or diphosphate, chloride, potassium, maleate, calcium, citrate, mesylate, nitrate, succinate, tartrate, aluminum, and gluconate. In one embodiment, for example, the escitalopram is an escitalopram oxalate.

The amount of each active agent of the invention that will be effective in the treatment of premature ejaculation can be determined by standard clinical techniques based on the present description. In addition, *in vitro* assays may optionally be employed to help identify optimal dosage ranges. The precise dose to be employed in the formulation will also depend on the route of administration, and the seriousness of the condition, and should be decided according to the judgment of the practitioner and each subject's circumstances. However, suitable dosage ranges for oral administration are generally about 35-500 micrograms of each active agent *(e.g.,* tadalafil and escitalopram) per kilogram body weight. Other suitable dosage ranges for oral administration are generally about 0.05-30 milligrams of each active agent *(e.g.,* tadalafil or tamsulosin, and escitalopram) per dose. Suitable dosage ranges for intranasal administration are generally about 0.01 pg/kg body weight to 1 mg/kg body weight. Effective doses may be extrapolated from dose- response curves derived from in vitro or animal model test systems.

For systemic administration, a therapeutically effective dose can be estimated initially from *in vitro* assays. For example, a dose can be formulated in animal models to achieve a circulating concentration range that includes the IC50 as determined *in vitro.* Such information can be used to more accurately determine useful doses in humans. Initial dosages can also be estimated from in vivo data, e.g., animal models, using techniques that are well known in the art. One having ordinary skill in the art could readily optimize administration to humans based on animal data.

Dosage amount and interval may be adjusted individually to provide plasma levels of the compounds that are sufficient to maintain therapeutic effect. A person having ordinary skill in the art will be able to optimize therapeutically effective local dosages without undue experimentation.

The amount of compound administered will, of course, be dependent on the subject being treated, on the subject's weight, the severity of the affliction, the manner of administration, and the judgment of the prescribing physician. The therapy may be repeated intermittently while symptoms are detectable or even when they are not detectable. The therapy may be provided alone or in combination with other drugs.

In a preferred formulation, of the claimed invention, approximately 9 mg of the PDE5i tadalafil (free form or salts) is combined with approximately 18 mg escitalopram (free form or salts, e.g., oxalate) and one or more pharmaceutically acceptable excipients.

According to certain embodiments of the invention, the pharmaceutical formulation is formulated as a formulation to be administered on-demand to a subject suffering from premature ejaculation or desiring to increase intravaginal ejaculatory latency time (IELT). In a specific formulation for on-demand administration, approximately 6.9 mg of the PDE5i tadalafil (free form or salts) is combined with approximately 9.6 mg escitalopram (free form or salts) and one or more pharmaceutically acceptable excipients. In another specific formulation for on-demand administration, approximately 6.9 mg of the PDE5i tadalafil (free form or salts) is combined with approximately 19.2 mg escitalopram (free form or salts) and one or more pharmaceutically acceptable excipients. In another specific formulation for on-demand administration, approximately 13.8 mg of the PDE5i tadalafil (free form or salts) is combined with approximately 19.2 mg escitalopram (free form or salts) and one or more pharmaceutically acceptable excipients. In another specific formulation for on-demand administration, approximately 5 mg of the PDE5i tadalafil (free form or salts) is combined with approximately 5 mg escitalopram (free form or salts) and one or more pharmaceutically acceptable excipients. In yet another specific formulation for on-demand administration, approximately 9 mg of the PDE5i tadalafil (free form or salts) is combined with approximately 18 mg escitalopram (free form or salts) and one or more pharmaceutically acceptable excipients.

Also disclosed, but not part of the claimed invention, is a specific formulation for on-demand administration, approximately 0.2 mg of the alpha- blocker tamsulosin (free form or salts) is combined with approximately 9.6 mg escitalopram (free form or salts) and one or more pharmaceutically acceptable excipients. Also disclosed is a specific formulation for on-demand administration, approximately 0.2 mg of the alpha-blocker tamsulosin (free form or salts) is combined with approximately 19.2 mg escitalopram (free form or salts) and one or more pharmaceutically acceptable excipients. Also disclosed is another specific formulation for on- demand administration, approximately 0.4 mg of the alpha-blocker tamsulosin (free form or salts) is combined with approximately 19.2 mg escitalopram (free form or salts) and one or more pharmaceutically acceptable excipients. Also disclosed is another specific formulation for on-demand administration, approximately 0.4 mg of the alpha-blocker tamsulosin (free form or salts) is combined with approximately 18 mg escitalopram (free form or salts) and one or more pharmaceutically acceptable excipients.

The excipients for each formulation depend in part on the route of administration (parenteral, nasal, buccal) and the desired properties of the formulation, such as extended release,rapid oral dissolution, enteric coating, comingled or bilayered agents, etc.

For orally disintegrating tablets, the tablet may comprise microcrystals of active agent (coated or uncoated), at least one disintegration agent and at least one other excipient, such as water-soluble component, water-insoluble component, viscosity enhancer, and/or gel forming component, and optionally a surfactant *(e.g.*, polysorbate). The other excipient may comprise a mixture of powders and compressible microparticles to facilitate tablet pressing.

For example, the disintegration agent may comprise 0.5% to 30%, 1 to 20%, 2 to 15% or 3 to 15% by weight of the mass of the tablet, and the soluble component, insoluble component, gel forming component, and/or viscosity enhancer may comprise 40 to 90% by weight of the mass of the tablet. Disintegration agents may include for example maize starch or modified starches, cross- linked polyvinyl pyrrolidone, sodium carboxymethylcellulose, sodium starch glycolate, crospovidone, and the like. Effervescent agents may also be incorporated into the formulation.

Useful soluble components include polyols, such as mannitol, xylitol, sorbitol, maltitol, and the like; and sugars, such as sucrose, lactose, maltose, and the like. Useful insoluble components include di- or tri-basic calcium phosphate, organic fillers *(e.g.,* microcrystalline cellulose, PVP), water insoluble lubricants (e.g., magnesium stearate, sodium stearyl fumarate, stearic acid or glyceryl behenate) and/or glidants (e.g., talc, silicon dioxide). Useful gelling, swelling, and viscosity enhancers include guar gum, xanthan gum, alginates, dextran, pectins, polysaccharides, sodium or calcium carboxymethylcellulose, hydroxypropylcellulose, hydroxypropylmethylcellulose, and the like.

In one embodiment, an orally dissolving tablet containing the PDEi and the SRI contains a rapid dissolve tablet base powder, micronized silica gel, polyethylene glycol 3350, acesulfame potassium, steviol glycosides, and flavorings. Rapid dissolve tablet base powders are well-known in the art are generally available through commercial vendors, such as: ZYDIS^{®} (Catalant, Inc., Swindon, UK), ORAL BASE A (Fagron, Inc., St. Paul, MN), MEDI- RDT BASE (Medisa Network, Inc., St. Laurent, QC), RDT-PLUS^{™} (PCCA, Houston, TX), andDISINTEQUIK^{™} ODT (Kerry, Beloit, WI).

**[Deleted]**

**[Deleted]**

**[Deleted]**

**[Deleted]**

**[Deleted]**

**[Deleted]**

The invention provides a pharmaceutical formulation of the invention wherein the smooth muscle relaxant is a PDE5i with a PDEI IC50 to PDE5 IC50 selectivity ratio of > 1000, where the formulation comprises tadalafil or a pharmaceutically acceptable salt thereof as a PDE5i.

The invention provides a pharmaceutical formulation of the invention wherein the smooth muscle relaxant is a PDE5i comprising a diketopiperazine moiety, where the formulation comprises tadalafil or a pharmaceutically acceptable salt thereof as a PDE5i.

[Deleted]

[Deleted]

The invention provides a pharmaceutical formulation of the invention wherein the smooth muscle relaxant is the PDE5i tadalafil present in a single dose unit at about 1-30 mg.

The invention provides a pharmaceutical formulation of the invention wherein the smooth muscle relaxant is the PDE5i tadalafil present in a single dose unit at about 4-20 mg.

The invention provides a pharmaceutical formulation of the invention wherein the smooth muscle relaxant is the PDE5i tadalafil present in a single dose unit at about 4 mg, about 5mg, about 6.9 mg, about 9mg, or about 13.8 mg.

The invention provides a pharmaceutical formulation of the invention wherein the SRI is escitalopram and optionally a SRI selected from the group consisting of citalopram, fluoxetine, paroxetine, sertraline, dapoxetine, seproxetine, zimelidine, and mesembrine.

[Deleted]

[Deleted]

[Deleted]

**[Deleted]**

**[Deleted]**

The invention provides a pharmaceutical formulation of the invention wherein the SRI escitalopram is present in a single dose unit at about 4-15 mg.

The invention provides a pharmaceutical formulation of the invention wherein the SRI escitalopram is present in a single dose unit at about 4 mg, about 5mg, about 9.6 mg, about 18 mg, or about 19.2mg.

The invention provides a pharmaceutical formulation of the invention wherein the smooth muscle relaxant is the PDE5i tadalafil and the SRI is the SSRI escitalopram and wherein (a) said PDE5i present at 4 mg per dose and said SSRI present at 4 mg per dose, (b) said PDE5i present at 6.9 mg per dose and said SSRI present at 9.6 mg per dose, (c) said PDE5i present at 6.9 mg per dose and said SSRI present at 19.2 mg per dose, (d) said PDE5i present at 13.8 mg per dose and said SSRI present at 19.2 mg per dose, (e) said PDE5i present at 9 mg per dose and said SSRI present at 18 mg per dose, (f) said PDE5i present at 5 mg per dose and said SSRI present at 5 mg per dose. In an formulation not part of the claimed invention, the smooth muscle relaxant is an αl-adrenergic receptor antagonist present at 0.4 mg per dose and the SRI is an SSRI present at 18mg per dose.

The invention provides a pharmaceutical formulation of the invention wherein the smooth muscle relaxant is tadalafil or a pharmaceutically acceptable salt thereof, and the SRI is escitalopram or a pharmaceutically acceptable oxalate salt form.

The invention provides a pharmaceutical formulation of the invention wherein the pharmaceutical formulation is in an orally disintegrating tablet dosage form.

The invention also provides a pharmaceutical formulation of the present invention for use in the treatment of premature ejaculation.

In one embodiment the SRI is escitalopram, an enantiomer thereof, or a salt thereof; and the smooth muscle relaxant is tadalafil, an enantiomer thereof, or a salt thereof.

The invention provides a pharmaceutical formulation of the present invention for use in a method for delaying the onset of ejaculation in a male subject wherein the SRI and the smooth muscle relaxant are administered less than four hours prior to engaging in sexual activity.

The invention provides a pharmaceutical formulation of the present invention for use in a method for delaying the onset of ejaculation in a male subject wherein the SRI and the smooth muscle relaxant are administered at the same time.

The invention provides a pharmaceutical formulation of the present invention for use in a method for delaying the onset of ejaculation in a male wherein the SRI and the smooth muscle relaxant are administered in a single pharmaceutical formulation.

The invention provides a pharmaceutical formulation of the present invention for use in a method for delaying the onset of ejaculation in a male subject wherein the SRI and the smooth muscle relaxant are administered orally, buccally, sublingually, intranasally, or parenterally

The invention provides a pharmaceutical formulation of the present invention for use in a method for delaying the onset of ejaculation in a male subject wherein SRI and smooth muscle relaxant are as defined in the appended claims and wherein (a) the SRI and the smooth muscle relaxant are each administered in an amount of about 1 mg to about 30 mg, , (b) about 9.6 mg of SSRI and about 6.9 mg of PDE5i are administered on-demand, (c) about 18 mg of SSRI and about 0.4 mg of al-adrenergic receptor inhibitor are administered on-demand, (d) about 19.2 mg of SSRI and about 6.9 mg of PDE5i are administered on-demand, (e) about 5 mg of SSRI and about 5 mg of PDE5i are administered or on-demand, (f) about 19.2 mg of SRI and about 13.8 mg of PDE5i are administered on-demand, or (g) about 18 mg of SSRI and about 9 mg of PDE5i are administered on-demand.

**[Deleted]**

### EXAMPLES

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only to the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

### Example 1: Rapid dissolve escitalooram/tadalafil tablets

The following powdered ingredients were combined: 2.75% (w/w) escitalopram oxalate USP 39 powder, 1.37%(w/w) tadalafil powder, 81.22% (w/w) rapid dissolve tablet base powder, 3.82% (w/w) micronized silica gel, 9.16% (w/w) polyethylene glycol 3350, 0.61% (w/w) acesulfame potassium powder, 0.46% steviol glycosides 95% powder, and 0.61% (w/w) raspberry powder flavor.

The powder was thoroughly mixed and pressed into a Rapid Dissolve Powder Mold (Torpac, Inc., Fairfield, NJ) with about 0.6 - 0.7 g of powder per tablet. The Rapid Dissolve Powder Mold bottom plate was then baked in a convection oven at 110° C for 30 minutes. Once cooled to room temperature the tablets are packaged in an amber round blister pack with appropriate labeling of ingredients. Each rapid dissolve tablet contains about 9 mg of tadalafil and about 18 mg escitalopram oxalate.

Reference Example 1: Rapid dissolve escitalopram/tamsulosin tablets, not part of the claimed subject-matter but useful for understanding the invention.

The following powdered ingredients were combined: 2.78% (w/w) escitalopram oxalate USP 39 powder, 0.06% (w/w) tamsulosin powder, 82.30% (w/w) rapid dissolve tablet base powder, 3.82% (w/w) micronized silica gel, 9.16% (w/w) polyethylene glycol 3350, 0.61% (w/w) acesulfame potassium powder, 0.46% steviol glycosides 95% powder, and 0.61% (w/w) raspberry powder flavor. The powder is thoroughly mixed and pressed into a Rapid Dissolve Powder Mold (Torpac, Inc., Fairfield, NJ) with about 0.6 - 0.7 g of powder per tablet. The Rapid Dissolve Powder Mold bottom plate is then baked in a convection oven at 110° C for 30 minutes. Once cooled to room temperature the tablets are packaged in an amber round blister pack with appropriate labeling of ingredients. Each rapid dissolve tablet will contain about 0.4 mg of tamsulosin and about 18 mg escitalopram oxalate.

### Example 2: Clinical test of tadalafil/escitalopram oxalate 9mg/18mg dosage

Patients were selected based upon their response to a self-administered questionnaire designed to evaluate key elements of premature ejaculation (PE), including (1) self-estimated intravaginal ejaculation latency time (IELT), (2) personal control, (3) levels of distress, and (4) interpersonal difficulty. The decision to initiate therapy for PE was at the discretion of a licensed physician. Exclusion criteria included use of phosphodiesterase-5 enzyme inhibitor, organic nitrates, antidepressant medications, antipsychotic medications, and agents with serotonergic effects. Patients were prescribed escitalopram/tadalafil (18/9 mg) orally disintegrating tablets (ODT) and instructed to use one ODT 2 to 24 hours prior to sexual intercourse, and no more than one ODT in a 24-hour period. The self-administered questionnaire was re-administered following the use of four ODTs.

A total of thirteen patients were included in this initial analysis. The median age was 36 years old (interquartile range (IQR) 26-40 years). The escitalopram/tadalafil combination improved IELT in 100% of patients. Moreover, there was a significant reduction in the number of patients reporting an IELT of 3 minutes or less post-treatment versus pre-treatment (0% vs. 69%; p<0.001). Additionally, significantly more patients reported a delay in IELT of greater than 10 minutes post-treatment compared to pre-treatment (69% vs. 0%; p<0.001). The majority of patients (69%) reported an improvement in composite patient-reported outcome measures (perceived control, personal distress, and interpersonal difficulty) post-treatment with escitalopram/tadalafil. More patients gave ratings of "very poor" or "poor" for control over ejaculation pre-treatment versus post-treatment (38% pre- vs. 8% post-treatment). More patients gave ratings of "very" or "extremely" for personal distress pre-treatment versus post-treatment (38% pre- vs. 15% post-treatment).

Ninety one percent of patients reported a noticeable improvement in sexual ability and/or performance after treatment with escitalopram/tadalafil and 77% of patients reported an improvement in overall satisfaction with sexual experience. Of the 13 patients analyzed, three reported a failure to ejaculate post-treatment. Common side effects reported included bad taste (8%, n=l), nausea/vomiting (8%, n=l), and headache (15%, n=2).

Overall, treatment with escitalopram/tadalafil was well tolerated and resulted in improvements of both IELT and patient-reported outcome measures in men with PE.

### Reference Example 2: Clinical test of tamulosin/escitalonram oxalate 0.4 mg/18 mg dosage, not part of the claimed subject-matter but useful for understanding the invention.

Patients are selected based upon their response to a self-administered questionnaire designed to evaluate key elements of premature ejaculation (PE), including (1) self-estimated intravaginal ejaculation latency time (IELT), (2) personal control, (3) levels of distress, and (4) interpersonal difficulty. The decision to initiate therapy for PE is at the discretion of a licensed physician. Exclusion criteria include use of alpha-blockers, phosphodiesterase-5 enzyme inhibitor, organic nitrates, antidepressant medications, antipsychotic medications, and agents with serotonergic effects. Patients are prescribed escitalopram/tamsulosin (18/0.4 mg) orally disintegrating tablets (ODT) and are instructed to use one ODT 2 to 24 hours prior to sexual intercourse, and no more than one ODT in a 24-hour period. The self-administered questionnaireis re-administered following the use of four ODTs.

## Claims

1. A pharmaceutical formulation for use within 36 hours before coitus, 2 hours to about 24 hours before coitus, or 30 minutes to about 5 hours before coitus to delay onset of ejaculation by a patient in need thereof comprising tadalafil, escitalopram, and one of more pharmaceutically acceptable excipients.

2. The pharmaceutical formulation of claim 1, wherein tadalafil is present in a single dose unit of the pharmaceutical formulation at 1-30 mg (± 2%); 4-20 mg (± 2%); 4 mg (± 2%), 5 mg (± 2%), 6.9 mg (± 2%), 9 mg (± 2%), or 13.8 mg (± 2%).

3. The pharmaceutical formulation of claim 1 or 2, wherein the escitalopram is present in a single dose unit at 1-30 mg (± 2%); 4-20 mg (± 2%); 4 mg (± 2%); 5 mg (± 2%); 9.6 mg (± 2%); 18 mg (± 2%); or 19.2 mg (± 2%).

4. The pharmaceutical formulation of claim 2 or 3, wherein the formulation is solid single dosage form comprising:
a. 4-15 mg (± 2%) tadalafil and 4-20 mg (± 2%) escitalopram;
b. 9 mg (± 2%) tadalafil and 18 mg (± 2%) escitalopram;
c. 5 mg (± 2%) tadalafil and 5 mg (± 2%) escitalopram;
d. 4 mg (± 2%) tadalafil and 4 mg (± 2%) escitalopram;
e. 6.9 (± 2%) mg tadalafil and 9.6 mg (± 2%) escitalopram;
f. 6.9 mg (± 2%) tadalafil and 19.2 mg (± 2%) escitalopram; or
g. 13.8 mg (± 2%) tadalafil and 19.2 mg (± 2%) escitalopram.

5. The pharmaceutical formulation of any one of claims 1-4, wherein use is within 36 hours prior to coitus.

6. The pharmaceutical formulation of any one of claims 1-5, wherein use is from 30 minutes to 5 hours prior to coitus.

7. The pharmaceutical formulation of any one of claims 1-6, wherein use is from 2 hours to 24 hours prior to coitus.

8. The pharmaceutical formulation of any one of claims 2, 3, and 5-9, wherein the formulation is a combined dosage form of the formulation where the concentration of each of escitalopram and tadalafil is 1 mg (± 2%) to 30 mg (± 2%) per dose.

9. The pharmaceutical formulation of any one of claims 1-8, wherein said pharmaceutical formulation is a combined dose formulation in the form of a tablet, a capsule, a liquid, a powder, a lyophilized cake, an oral film, troche, or an intranasal preparation.

10. The pharmaceutical formulation of any one of claims 1-8, wherein said pharmaceutical formulation is a rapid dissolve tablet.

## Patentansprüche

1. Pharmazeutische Formulierung für die Verwendung innerhalb von 36 Stunden vor dem Koitus, 2 Stunden bis etwa 24 Stunden vor dem Koitus oder 30 Minuten bis etwa 5 Stunden vor dem Koitus für die Verzögerung des Beginns der Ejakulation durch einen Patienten, der dies benötigt, umfassend Tadalafil, Escitalopram und einen oder mehrere pharmazeutisch unbedenkliche Hilfsstoffe.

2. Pharmazeutische Formulierung nach Anspruch 1, wobei Tadalafil in einer einzelnen Dosiseinheit der pharmazeutischen Formulierung bei 1-30 mg (± 2 %); 4-20 mg; 4 mg (± 2 %); 5 mg (± 2 %); 6,9 mg (± 2 %); 9 mg (± 2 %); oder 13,8 mg (± 2 %) vorhanden ist.

3. Pharmazeutische Formulierung nach Anspruch 1 oder 2, wobei das Escitalopram in einer Einzeldosiseinheit bei 1-30 mg (± 2 %); 4-20 mg (± 2 %); 4 mg (± 2 %); 5 mg (± 2 %); 9,6 mg (± 2 %); 18 mg (± 2 %); oder 19,2 mg (± 2 %) vorhanden ist.

4. Pharmazeutische Formulierung nach Anspruch 2 oder 3, wobei die Formulierung eine feste Einzeldosierungsform ist, umfassend:
a. 4-15 mg (± 2 %) Tadalafil und 4-20 mg (± 2 %) Escitalopram;
b. 9 mg(± 2 %) Tadalafil und 18 mg (± 2 %) Escitalopram;
c. 5 mg(± 2 %) Tadalafil und 5 mg (± 2 %) Escitalopram;
d. 4 mg(± 2 %) Tadalafil und 4 mg (± 2 %) Escitalopram;
e. 6,9 (± 2 %) mg Tadalafil und 9,6 mg (± 2 %) Escitalopram;
f. 6,9 mg(± 2 %) Tadalafil und 19,2 mg (± 2 %) Escitalopram; oder
g. 13,8 mg (± 2 %) Tadalafil und 19,2 mg (± 2 %) Escitalopram.

5. Pharmazeutische Formulierung nach einem der Ansprüche 1-4, wobei die Verwendung innerhalb von 36 Stunden vor dem Koitus erfolgt.

6. Pharmazeutische Formulierung nach einem der Ansprüche 1-5, wobei die Verwendung innerhalb von 30 Minuten bis 5 Stunden vor dem Koitus erfolgt.

7. Pharmazeutische Formulierung nach einem der Ansprüche 1-6, wobei die Verwendung innerhalb von 2 Stunden bis 24 Stunden vor dem Koitus erfolgt.

8. Pharmazeutische Formulierung nach einem der Ansprüche 2, 3 und 5-9, wobei die Formulierung eine kombinierte Dosierungsform der Formulierung ist, wobei die Konzentration jeweils von Escitalopram und Tadalafil 1 mg (± 2 %) bis 30 mg (± 2 %) pro Dosis beträgt.

9. Pharmazeutische Formulierung nach einem der Ansprüche 1-8, wobei die pharmazeutische Formulierung eine kombinierte Dosisformulierung in der Form einer Tablette, einer Kapsel, einer Flüssigkeit, eines Pulvers, eines lyophilisierten Kuchens, einer oralen Folie, einer Pastille oder eines intranasalen Präparats ist.

10. Pharmazeutische Formulierung nach einem der Ansprüche 1-8, wobei die pharmazeutische Formulierung eine schnell lösliche Tablette ist.

## Revendications

1. Préparation pharmaceutique pour l'utilisation au sein d'une période de 36 heures avant le coït, 2 heures à environ 24 heures avant le coït, ou 30 minutes à environ 5 heures avant le coït pour retarder la survenue d'éjaculation par un patient en ayant besoin, comprenant du tadalafil, de l'escitalopram, et un ou plusieurs excipients pharmaceutiquement acceptables.

2. Préparation pharmaceutique selon la revendication 1, dans laquelle le tadalafil est présent en unité unidose de la préparation pharmaceutique à 1 à 30 mg (± 2%) ; 4 à 20 mg (± 2%) ; 4 mg (± 2%), 5 mg (± 2%), 6,9 mg (± 2%), 9 mg (± 2%), ou 13,8 mg (± 2%).

3. Préparation pharmaceutique selon la revendication 1 ou 2, dans laquelle l'escitalopram est présent en unité unidose à 1 à 30 mg (± 2 %) ; 4 à 20 mg (± 2 %) ; 4 mg (± 2 %) ; 5 mg (± 2 %) ; 9,6 mg (± 2 %) ; 18 mg (± 2 %) ; ou 19,2 mg (± 2 %).

4. Préparation pharmaceutique selon la revendication 2 ou 3, dans laquelle la préparation est en forme d'unidose solide, comprenant :
a. 4 à 15 mg (± 2 %) de tadalafil et 4 à 20 mg (± 2 %) d'escitalopram ;
b. 9 mg (± 2 %) de tadalafil et 18 mg (± 2 %) d'escitalopram ;
c. 5 mg (± 2 %) de tadalafil et 5 mg (± 2 %) d'escitalopram ;
d. 4 mg (± 2 %) de tadalafil et 4 mg (± 2 %) d'escitalopram ;
e. 6,9 (± 2 %) mg de tadalafil et 9,6 mg (± 2 %) d'escitalopram ;
f. 6,9 mg (± 2 %) de tadalafil et 19,2 mg (± 2 %) d'escitalopram ; ou
g. 13,8 mg (± 2 %) de tadalafil et 19,2 mg (± 2 %) d'escitalopram.

5. Préparation pharmaceutique selon l'une quelconque des revendications 1 à 4, dans laquelle l'utilisation est au sein d'une période de 36 heures avant le coït.

6. Préparation pharmaceutique selon l'une quelconque des revendications 1 à 5, dans laquelle l'utilisation est de 30 minutes à 5 heures avant le coït.

7. Préparation pharmaceutique selon l'une quelconque des revendications 1 à 6, dans laquelle l'utilisation est de 2 heures à 24 heures avant le coït.

8. Préparation pharmaceutique selon l'une quelconque des revendications 2, 3, et 5 à 9, dans laquelle la préparation est en forme de dose combinée de la préparation où la concentration de chacun d'escitalopram et de tadalafil est de 1 mg (± 2 %) à 30 mg (± 2 %) par dose.

9. Préparation pharmaceutique selon l'une quelconque des revendications 1 à 8, dans laquelle ladite préparation pharmaceutique est une préparation en dose combinée sous la forme d'un comprimé, d'une capsule, d'un liquide, d'une poudre, d'un gâteau lyophilisé, d'un film oral, d'une pastille, ou d'une formule intranasale.

10. Préparation pharmaceutique selon l'une quelconque des revendications 1 à 8, dans laquelle ladite préparation pharmaceutique est un comprimé à dissolution rapide.
